# EUROPEAN PATENT APPLICATION

(11) **EP 4 122 916 A1**
(43) Date of publication of application: **25.01.2023**
(21) Application number: 21187217.1
(22) Date of filing: 22.07.2021
(51) Int. Cl.: C07D 209/16, A61K 31/4045, A61P 25/26

(54) **METHOD FOR PREPARING AN ORGANIC COMPOUND**

(71) Applicant: GH Research Ireland Limited, Dublin 2 (IE)
(72) Inventor: Chubb, Richard, Sunderland, SR5 2TQ (GB); Matters, Rebecca, Sunderland, SR5 2TQ (GB)
(74) Representative: Breuer, Markus

(57) **Abstract**

A method for preparing 5-methoxy-N,N-dimethyltryptamine (5-MeO-DMT) is provided which starts from 5-methoxy-1H-indole. The product obtained can be purified via formation of a salt, such as a fumarate salt, of 5-MeO-DMT.

## Description

### Technical Field

The present invention relates to a method for preparing an organic compound, specifically 5-Methoxy-N,N-dimethyltryptamine (5-MeO-DMT). The invention furthermore relates to the purification of the prepared 5-MeO-DMT.

### Background

5-Methoxy-N,N-dimethyltryptamine (5-MeO-DMT) has the formula shown below.

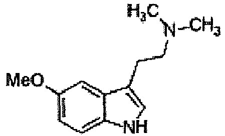

5-MeO-DMT is a naturally occurring serotonergic psychedelic tryptamine which acts as a 5-HT1A and 5-HT2A receptor agonist.

5-MeO-DMT is synthesized in human pineal and retina, and it has been found in human body fluids including urine, blood, and cerebrospinal fluid.

5-MeO-DMT was first isolated from the bark of *Dictyoloma incanescens,* but it is also contained in other plants, and it has been identified as the major active ingredient in the venom of *Bufo alvarius* toads.

The chemical synthesis of 5-MeO-DMT has been described in 1936 by Hoshino and Shimodaira (Bulletin of the Chemical Society of Japan, 11(3), 221-224). No medical or other use is contemplated in this publication.

According to Hoshino and Shimodaira, following a reaction between 5-methoxy indolyl 3-ethyl beta bromide and dimethyl amine the product is isolated and purified by distillation under reduced pressure. It is also reported that the substance crystallises from ether-petrol ether. No conditions are disclosed.

The product obtained is described as nice colorless prisms having a melting point of 66-67°C. There is no characterisation regarding the amounts of impurities contained in the product.

A comparison of the reported melting point with later data regarding the melting point of 5-MeO-DMT (69-70°C) may, however, be taken as an indication that impurities are still present.

Moreover, given the high boiling point of 5-MeO-DMT even under reduced pressure (208 to 210°C at 4 mm), distillation is not an advantageous purification method.

Somei et al. (Chem. Pharm. Bull. 49(1) 87 - 96 (2001)) report syntheses of serotonin, N-methylserotonin, bufotenine, 5-methoxy-N-methyltryptamine, bufobutanoic acid, N-(indol-3-yl)methyl-5-methoxy-N-methyltryptamine, and lespedamine.

In the context of a synthesis for bufotenine, a mixture of compounds comprising 5-MeO-DMT is obtained from which the components are purified by column chromatography. 5-MeO-DMT is then recrystallised from Et₂O-hexane. Details regarding the recrystallisation conditions or the amounts of impurities contained in the product are not disclosed. The liquid mixture used for recrystallisation (Et₂O-hexane) is similar to the mixture used by Hoshino and Shimodaira (ether-petrol ether).

A.M. Sherwood et al. (ACS Omega 2020, 5, 49, 32067-32075) report on synthetical methods to obtain salts of 5-MeO-DMT.

Based on its physiological activities, there has recently been an interest in potential medical uses of 5-MeO-DMT, for instance, investigating potential medical uses in human clinical trials.

For such uses in human clinical trials, and for potential use in an approved medical product, 5-MeO-DMT in sufficient quantities and in high purity is required. For administration to humans, purity as high as possible is necessary. According to the invention, it is in particular desirable that the total amount of impurities in the drug substance is below 0.5% and that the amount of each individual impurity is below 0.1%.

Furthermore, limits as regards the amount of residual solvent are to be observed.

Against this background, there is a need for providing a simple method of purifying 5-MeO-DMT, in particular of purifying 5-MeO-DMT so as to obtain the substance in a pharmaceutical grade. There is also a need for providing a 5-MeO-DMT in a form meeting specific purity requirements.

### Summary of the Invention

The present invention relates to a method for preparing 5-methoxy-N,N-dimethyltryptamine (5-MeO-DMT) comprising
converting 5-methoxy-1H-indole into a ketoamide 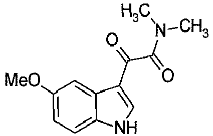 and reducing this ketoamide to obtain 5-MeO-DMT, wherein the 5-methoxy-1H-indole is added to oxalyl chloride and the acid chloride intermediate obtained is reacted with dimethylamine.

The intermediates and in particular the final product are obtained in high purity.

Specific embodiments are defined in the dependent claims.

### Detailed Description of the Invention

The present invention is based on the discovery that 5-methoxy-N,N-dimethyltryptamine (5-MeO-DMT) can be obtained in high purity suitable for pharmaceutical use based on commercially available starting materials using a scalable process.

The first stage of the synthetic route according to the present invention involves the reaction of 5-methoxy-1H-indole, a commercially available compound, with oxalyl chloride, which is also commercially available. The obtained acid chloride intermediate is then reacted with dimethylamine to obtain a ketoamide.

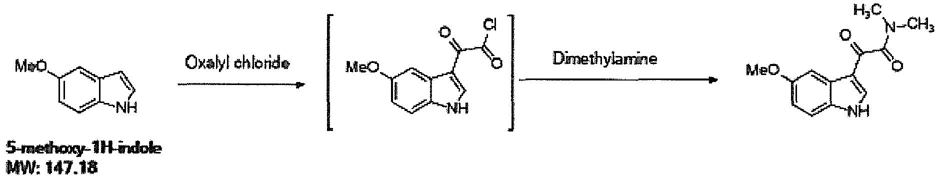

According to the invention, 5-methoxy-1H-indole is added to a solution of oxalyl chloride to increase the yield of the acid chloride intermediate and to supress the formation of side products.

The oxalyl chloride is preferably used in a molar excess, relative to the 5-methoxy-1H-indole, for instance, 1.05 to 2 eq, preferably 1.1 to 1.5 eq, in particular about 1.2 eq.

The reaction is preferably carried out in a solvent.

To this end, a mixture of oxalyl chloride and the solvent or solvent combination employed is prepared.

Suitable solvents are, for instance, t-butyl methyl ether (TBME), tetrahydrofuran (THF) and mixtures thereof. Preferred are mixtures of containing TBME and THF in ratio of 10:1 - 1:1 (vol:vol), in particular TBME/THF (6:1) (vol:vol).

The reaction is preferably carried out at an elevated temperature, such as a temperature in the range of 35-45 °C. Preferably, 5-methoxy-1H-indole is added to a solution of oxalyl chloride having a temperature within this range.

The 5-methoxy-1H-indole is preferably added in the form of a solution, in particular a solution in a combination of t-butyl methyl ether and tetrahydrofuran. The solution may be added as a steady stream within, for instance, 15-30 minutes, whilst maintaining the temperature 35-45°C.

Preferably, the reaction mixture is then stirred at 35-45°C for up to 2 h such as for 30 minutes.

The acid chloride intermediate can then be isolated and optionally purified, applying conventional methods.

Preferably, however, the acid chloride intermediate is subjected to a reaction with dimethylamine without prior isolation.

The reaction of the acid chloride intermediate with dimethylamine is preferably carried out at a temperature between 0-25°C.

The dimethylamine is preferably used in a molar excess relative to the acid chloride intermediate, for instance, 2 to 6 eq., preferable3 to 5 eq., in particular about 4.25 eq.

The amount can be adjusted based on the pH of the reaction mixture following addition. If the pH is <7, additional dimethylamine can be added until a pH of >7 is achieved.

The dimethylamine is preferably added as a solution in an inert solvent, such as tetrahydrofuran. For instance, a 2M solution can be used. Such a solution can be added dropwise whilst maintaining the temperature. The addition time can be between 0.5 and 2 h, such as about 1 h.

After completion of the addition, the reaction mixture is preferably stirred at 15-25°C for 1.5-2.5 h, such as about 2 h.

The ketoamide is isolated from the resulting mixture.

Typically, the resulting suspension is filtered.

The filter cake may then be washed, for instance, with pre-mixed t-butyl methyl ether and tetrahydrofuran, then twice with n-heptane and pulled dry.

For further purification, solid material recovered from the above reaction mixture, for instance, by the method outlined, is treated with a solvent to remove side products. In a preferred embodiment, water (8.0 mL/g) is added to, and the resulting suspension is stirred at 15-25°C for at least 1.5 h.

Alternative solvent mixtures with an organic component (10-25% acetone, MeOH or MeCN) are also effective.

To recover the product, the suspension is filtered, and the filter cake is washed and pulled dry.

Preferably, the solids are then dried in a vacuum oven at 60°C until no further weight loss.

Stage 1 typically achieves a yield of >90% in excellent purity (>97%).

Stage 2 of the synthetic route according to the present invention involves reducing the ketoamide obtained in stage 1.

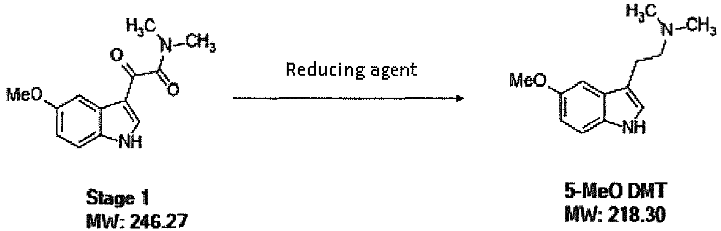

The ketoamide obtained in stage 1 is reacted with a reducing agent. Preferably, the reaction is carried out in solution. After the reaction, the resulting mixture is subjected to a workup to isolate the product and to remove oxidised as well as unreacted reducing agent.

In a preferred embodiment, the reducing agent is lithium aluminium hydride.

To reduce the ketoamide using this agent, the ketoamide is first combined with an inert solvent. Preferred solvents are tetrahydrofuran, methyl tetrahydrofuran and 1,4-dioxane. The resulting solution or suspension typically is cooled, with stirring, to 0-10°C.

The reducing agent lithium aluminium hydride is used in an amount, relative to the ketoamide, of 2 to 6 eq, preferably about 2.5 eq.

Lithium aluminium hydride is added as a preparation in an inert solvent, such as THF.

in a preferred embodiment, 2.4 M lithium aluminium hydride in THF (2.5eq) is added dropwise whilst maintaining the reaction temperature between 0 to 25°C.

The resulting suspension is preferably warmed to 15 to 25°C and stirred for 30 to 60 minutes, followed by heating to 60 to 95°C, preferably 75 to 80°C, and stirring for 8 to 24 hours, such as 12 to 18 hours.

Subsequently, the reaction is worked up by quenching with acetone followed by 20% citric acid in water. The reaction temperature is typically between 0-30°C. The Li-AI byproducts are then filtered off.

Preferably, the filter cake is resuspended in reaction solvent, such as THF, and stirred for 8 to 24 hours, such as 12 to 18 hours. The suspension is then subjected to a further filtration. The filtrate is combined with the filtrate obtained above.

The combined filtrates are evaporated at a maximum temperature 50°C.

The crude product thus obtained may contain several impurities, such as the side products indicated by the formula shown below.

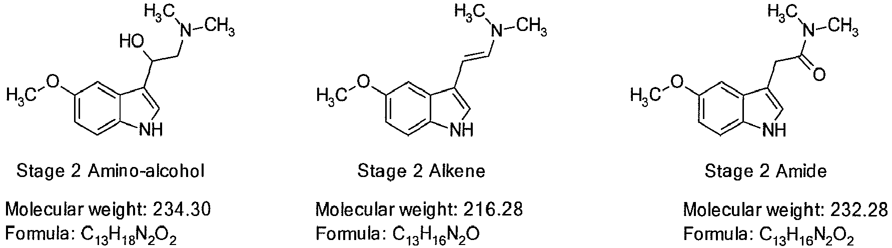

In a preferred embodiment, the crude product obtained is purified by column chromatography using, for instance, a column containing silica as stationary phase and a gradient elution with 5-15% methanol in ethyl acetate.

Product containing eluate fractions are combined and evaporated to dryness at a maximum temperature of 50°C. Subsequently, the product is dried under vacuum at 40°C.

The chromatography can lead to a complete removal of the amino-alcohol impurity.

A purity of ≥ 97.5% can be achieved.

Optional stage 3 of the synthetic route according to the present invention increases the purity of the 5-MeO-DMT. It involves
(a) reacting the 5-MeO-DMT obtained with an acid;
(b) optionally purifying the acid addition salt formed;
(c) subsequent salt break;
(d) isolating 5-MeO-DMT.

Crude 5-MeO-DMT is combined with a solvent. Suitable solvents are, for instance, ethanol, isopropanol, acetone, isopropyl acetate. A preferred solvent is ethanol.

A suitable acid to form an acid addition salt with 5-MeO-DMT is then added.

Preferably, the acid is fumaric acid.

The acid is preferably added in a stochiometric amount necessary to form the intended salt.

The molar ratio 5-MeO-DMT:acid is preferably 1:1.

The acid can be added neat or combined with a solvent.

The mixture is then heated, under stirring, to 30 to 100°C, typically for 10 minutes to 3 hours. Preferably, a solution is obtained.

The mixture is then cooled to a temperature in the range of -10 to 25°C and kept at the reduced temperature to allow formation of a precipitate.

The mixture is then filtered. The filter cake is recovered.

Optionally, the recovered material is subjected to a re-crystallisation step, preferably using a solvent different from the solvent used for the formation of the salt.

The recovered solids are optionally dried.

The salt prepared, optionally after further purification, is then subjected to a salt break step.

To release 5-MeO-DMT from the salt, it is reacted with a base.

The base is, for instance, a hydroxide, a carbonate, a hydrogen carbonate or ammonia. The base is in particular selected from sodium hydroxide, potassium hydroxide, ammonia, sodium carbonate, sodium hydrogen carbonate, potassium carbonate, potassium hydrogen carbonate, and ammonia. Preferred bases are sodium carbonate and potassium carbonate.

The base can be added in the form of an aqueous solution.

After or preferably before adding the base, a solvent is added. The solvent is not miscible with water. 5-MeO-DMT in free base form released from the salt will dissolve in the solvent. A suitable solvent is an ether, in particular TBME.

If necessary, water is added.

The mixture is stirred for 10 minutes to 3 hours.

The aqueous phase and the organic phase are separated. The aqueous phase is extracted with an organic solvent, in particular the same solvent as used for the reaction mixture, and then discarded.

The combined organic phases are dried, for instance, with MgSO₄, and concentrated in vacuo (bath temperature of, for instance, 40 °C). The product obtained is further dried under vacuum, for instance, at 40 °C.

Purification of 5-MeO DMT *via* formation of a salt and subsequent salt break is able to provide high purity material (>99.5%) in good yield.

Preferably, the amount of each individual impurity is below 0.1%.

The total weight amount of solvents in the product is NMT 5000 ppm, preferably NMT 2500 ppm.

The following examples are intended to further illustrate the invention.

### Examples

### Example 1 - Preparation of the ketoamide intermediate

To a 10 L flange flask was added TBME (643 mL) and THF (108 mL). Oxalyl chloride (171.7 mL, 2.04 mol) was then added in one portion (exotherm up to 21.3 °C). The solution was heated to 35-45 °C and a solution of 5-methoxyindole (250 g, 1.70 mol) in TBME/THF (1.5 L/250 mL) was added as a steady stream over 30 minutes (exotherm up to 45.2 °C). A bright orange solid precipitated ∼1/4 of the way through the addition (2.1 °C exotherm). The reaction was stirred for 30 minutes at 35-45 °C then HPLC analysis (DMT, H₂O/MeCN or LGRAD) showed no indole remaining. The reaction was then cooled to 0-10 °C then dimethylamine (2M in THF, 7.22 mol, 3.61 L) was added as a steady stream over 1h 20 minutes (exotherm up to 22 °C). The pH was measured at pH7 then the resulting thick, beige slurry was warmed to RT and stirred for 1 hour. HPLC analysis (DMT, H₂O/MeCN) showed no acid chloride intermediate remaining. The slurry was filtered and the filter cake was washed with TBME/THF (855 mL/145 mL) then heptane (2 x 1 L). The solid was then dried under vacuum at 60 °C overnight.

The crude solid was then charged to a 5 L flange flask and slurried in water (2 L) for 2 h. This was then filtered and washed with water (500 mL) then dried under vacuum at 60 °C with a bleed and a water trap for 2 days. This gave 393.7 g (94% yield) which had an HPLC purity of 99.6% and a water content by KF of 0.38%.

### Example 2 - Preparation of the 5-MeO-DMT

To a 10 L flange flask was added Stage 1 (318 g, 1.29 mol) and 2-Me THF (4388 mL) - beige suspension formed. This was cooled to 0-10 °C and LiAlH₄ (2.4 M in THF, 3.23 mol, 1345 mL) was added dropwise over 1 hour (gas evolution, exotherm, yellow slurry formed). The reaction was then warmed to RT and stirred for 30 minutes before it was heated to 75-80 °C (bright yellow solution formed). The reaction was stirred for 18 h and HPLC analysis showed no Stage 1 remaining with 82.0% Stage 2, 11.2% amino-alcohol intermediate and 3.4% alkene intermediate. The reaction was cooled to 0-10 °C then acetone (905 mL) was added dropwise (gas evolution, exotherm) followed by a 20% citric acid solution (481 mL) dropwise (gas evolution, exotherm). The resulting beige suspension was warmed to RT and stirred for 1 hour. It was then filtered and the solids were washed with THF (640 mL). The solids were then returned to the flask and slurried in THF (6.7 L) for 18 h. The suspension was filtered and the solids washed with THF (1590 mL) and the two filtrates were combined and concentrated *in vacuo* to give an orange oil (293.6 g, 271.6 g active, 96% crude yield).

The crude was combined with that from other reactions (560 g total crude Stage 2 input) and purified by column chromatography (10 eq. SiO₂, 5-15% MeOH in EtOAc, TLC system: 20% MeOH/EtOAc). The product was present in fractions 10-19 and 20-26 which were combined and concentrated *in vacuo* to give two portions of a light brown solid giving 251 g which had an HPLC purity of 98.4% and 120 g (371 g total, 1.70 mol, 66%) which had an HPLC purity of 97.5%.

The above materials were charged to a 5 L flange flask along with 5.02 g of crude material and 3.5L TBME was added. The mixture was heated to 35-40 °C and a brown solution was formed. The mixture was then concentrated *in vacuo* then further dried under vacuum at 40 °C to give 369.1 g of a light brown solid which had an HPLC purity of 98.4%.

### Example 3 - Purification of the 5-MeO-DMT via Salt Formation

To a 2L jacketed vessel was added 5-MeO DMT (100 g, 0.46 mol) and ethanol (800 mL). Fumaric acid (53.2 g, 0.46 mol) was then added in one portion - 2 °C exotherm. The resulting suspension was heated to 70-75 °C and a brown solution was formed. The reaction was held at 70-75 °C for 30 minutes then cooled to 0-5 °C over 1 hr. The resulting suspension was filtered and the cake was washed with ethanol (200 mL). The solid was dried under vacuum overnight at 50 °C to give 133.4 g of 5-MeO DMT fumarate salt (1:1) which had an HPLC purity of 99.8%.

The above fumarate was charged to a 2L jacketed vessel and TBME (1330 mL) was added followed by 1M potassium carbonate (665 mL). The initial light brown suspension formed a solution after 2 minutes of stirring at RT. The reaction mixture was stirred for 1 hour then the phases were separated. The aqueous was extracted with TBME (665 mL) and the combined organic phases were dried with MgSO₄, filtered and concentrated *in vacuo.* The damp solid was further dried under vacuum at 40 °C to give 82.3 g of a beige solid which had an HPLC purity of 99.8%.

## Claims

1. Method for preparing 5-methoxy-N,N-dimethyltryptamine (5-MeO-DMT) comprising converting 5-methoxy-1H-indole into a ketoamide 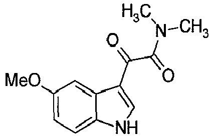 and reducing this ketoamide to obtain 5-MeO-DMT, wherein
the 5-methoxy-1H-indole is added to oxalyl chloride and the acid chloride intermediate obtained is reacted with dimethylamine.

2. Method according to claim 1, wherein the reaction between 5-methoxy-1H-indole and oxalyl chloride is carried out using a mixed solvent containing t-butyl methyl ether (TBME) and tetrahydrofuran (THF).

3. Method according to claim 1 or 2, wherein the acid chloride intermediate formed by the reaction between 5-methoxy-1H-indole and oxalyl chloride is not isolated.

4. Method according to any of the preceding claims, wherein the ketoamide is obtained in a purity >97%.

5. Method according to any of the preceding claims, wherein the ketoamide intermediate 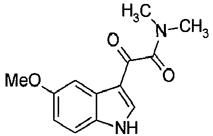 is reduced using lithium aluminium hydride.

6. Method according to claim 5, wherein the crude product of the reduction is purified by column chromatography using silica as stationary phase and a gradient elution with 5-15% methanol in ethyl acetate to obtain 5-MeO-DMT in a purity of ≥ 97.5%.

7. Method according to any of the preceding claims, wherein the method comprises the additional steps of
(a) reacting the 5-MeO-DMT obtained with an acid;
(b) optionally purifying the acid addition salt formed
(c) subsequent salt break;
(d) isolating 5-MeO-DMT.

8. Method according to claim 7, wherein the acid is fumaric acid.

9. Method according to any of claims 7 or 8, wherein the molar ratio 5-MeO-DMT : acid is 1:1.

10. Method according to any of claims 7 to 9, wherein 5-MeO DMT is obtained in a purity of >99.5%.

11. Method according to any of claims 7 to 10, wherein the amount of each individual impurity is below 0.1%.
